# EUROPÄISCHE PATENTANMELDUNG

(11) **EP 0 938 865 A1**
(43) Veröffentlichungstag der Anmeldung: **01.09.1999**
(21) Anmeldenummer: 99103722.7
(22) Anmeldetag: 25.02.1999
(51) Int. Cl.: A61B 5/0215

(54) **Einrichtung zur intermittierenden Messung des zentralvenösen Druckes**

(30) Priorität: 27.02.1998 DE 29803439 U
(71) Anmelder: CareMed Medical Produkte Aktiengesellschaft, 01109 Dresden (DE)
(72) Erfinder: Reuter, Jürgen, Dipl.-Ing., 36211 Alheim (DE); Gerlach, Roland, Dr.-Ing., 34302 Guxhagen (DE); Pfleiderer, Klaus, 60433 Frankfurt/Main (DE)

(57) **Zusammenfassung**

Die Erfindung betrifft eine Einrichtung zur intermittierenden Messung des zentralvenösen Druckes bei gleichzeitiger Einleitung von Infusionsflüssigkeiten in die obere Hohlvene des Menschen. Sie ist gekennzeichnet durch eine Druckmeßdose, die aus zwei Halbschalen besteht, die durch eine sehr weiche Membran getrennt sind. Durch die eine Halbschale fließt die Infusionsflüssigkeit, während die zweite Halbschale eine kugelabschnittförmige Wand mit einem zentralen Schlauchanschlußstutzen aufweist, über den ein elektronisches Meßinstrument mit digitaler Druckanzeige und einer Lichtmarkierungseinrichtung angeschlossen ist, die eine einfache Justierung auf die mittlere Axillarlinie des Patienten ermöglicht. Die erfindungsgemäße Druckmeßdose ist am deutlichsten in Fig. 2 dargestellt.

## Beschreibung

Die Messung des zentralvenösen Druckes (ZVD) liefert besonders bei Menschen auf der Intensivstation diagnostisch und therapeutisch wichtige und in vielen Fällen entscheidende Informationen. Zur Messung des ZVD wird dem Patienten über die V. jugularis, die V. subclavia oder die V. basilica ein dünner Katheter in die obere Hohlvene vorgeschoben. Aus den registrierten Druckwerten können Schlüsse gezogen werden auf akuten oder chronischen:
- Volumenmangel (z.B. Schock, Blutungen, etc.)
- Volumenüberbelastung (z.B. Langzeitinfusionstherapie)
- Herzinsuffizienz (z.B. Lungenembolie, Herzinfarkt, etc.).

### Stand der Technik

Die am meisten angewandte Methode ist die flüssigkeitsmanometrische Druckmessung. Dazu wird in die Flüssigkeitsleitung von dem Infusionsbehälter zum Patienten ein Drei-Wege-Hahn integriert. Der freie Anschluß des Hahnes wird mit einer Steigleitung verbunden, die an ihrem freien Ende mit einem bakteriendichten Filter versehen ist. Über die laufende Infusion wird die Steigleitung mit isotoner Flüssigkeit gefüllt und nach Verschluß der Infusion stellt sich innerhalb einer Ausgleichszeit ein Druckgleichgewicht zwischen dem venösen Druck in der Hohlvene und der Flüssigkeitshöhe in dem Steigrohr ein, die nur von der Atmung und eventuellen Patientenbewegungen überlagert ist. Nachteil dieser Methode ist die lange Zeit, die das Pflegepersonal bis zum Gleichgewichtszustand gebunden ist. Die Ablesegenauigkeit der Flüssigkeitssäule ist begrenzt. Die Gefahr einer Kontamination des Patienten durch das "bakteriendichte" Filter ist nicht sicher auszuschließen.

Eine andere Methode der Venendruckmessung, die jedoch selten Anwendung findet, stellt die "Tip-Katheter"-Messung dar. Der Patient wird mit einem Venenkatheter katheterisiert, an dessen Spitze sich ein Mikro-Druckwandler befindet. Das elektrische Meßsignal wird einem Verstärker und Anzeigegerät zugeführt. Nach Unterbrechung der Infusion ist innerhalb kurzer Zeit eine Druckmessung möglich. Nachteil dieser Methode ist der hohe Preis und die enorme Störanfälligkeit bzw. die Beschädigung des Tip-Katheters. Zusätzlich stellt die hygienische Reinigung des Katheters vor Wiederverwendung ein Problem dar.

Als Beispiele für die zum Stand der Technik gehörenden Einrichtungen seien das System Medifix der Fa. B. Braun Melsungen AG (Firmenprospekt Nr. 0603 0300 B.03.05.93/1), die Einrichtung gemäß DE-GM 89 14 252 und die Einrichtung gemäß DE-OS 17 66 926 genannt.

### Aufgabe der Erfindung

Aufgabe der Erfindung ist die Schaffung einer einfachen elektro-mechanischen Meßeinrichtung, die hohe Ablesegenauigkeit gewährleistet, Lufteinschlüsse vermeidet, ein schnelles Ablesen des Druckwertes ermöglicht, für den Patienten die hygienischen Verhältnisse verbessert und preiswert ist.

### Erfindungsgemäße Lösung der Aufgabe

Die Aufgabe wird dadurch gelöst, daß erfindungsgemäß in eine Infusionsleitung eine Druckmeßdose integriert ist. Die Druckmeßdose besteht aus zwei Halbschalen mit einer dazwischen angeordneten sehr weichen Membran. Diese Membran trennt die Druckmeßdose in einen Infusionsraum und einen Luftraum. Die Halbschale der Druckmeßdose, in der sich die Luft befindet, weist eine kugelabschnittartige Wölbung auf. Zentral in der Wölbung ist ein Schlauchanschlußstutzen vorgesehen. Vorteilhafterweise ist an der Druckmeßdose über dem Schlauchanschlußstutzen und einem zwischengeschalteten Schlauch ein elektronischer Druckwandler mit digitaler Anzeige angeschlossen. Zur Justierung der Druckmeßdose auf das Höhenniveau der Hohlvene des Patienten, sind die Druckmeßdose, der elektronische Druckwandler mit der digitalen Anzeige und eine einen markierenden Lichtstrahl erzeugende Lichtquelle, beispielsweise eine an sich bekannte Laserlichtquelle, vorteilhafterweise zu einer mechanischen Einheit verbunden. Um den Druck in der Infusionsleitung während des Zeitpunktes der Messung des zentralvenösen Druckes zu neutralisieren, ist oberhalb der Druckmeßdose eine Schiebeklemme in die Infusionsleitung eingesetzt.

### Vorteile der Erfindung

Die Erfindung besteht aus einem Einmalartikel und eines sehr einfachen allgemein bekannten Druckwandlers mit Anzeigevorrichtung. Der Einmalartikel ist in das Infusionssystem integriert und damit für jeden Patienten neu und steril. Eine Kommunikation der Infusionsflüssigkeit mit der Umgebung ist ausgeschlossen. Damit sind die hygienischen Bedingungen für den u. U. schwerstkranken Patienten optimal. Meßwertverfälschende Lufteinschlüsse im Meßschenkel sind ausgeschlossen. Die Messung kann sehr schnell erfolgen, eine genaue und eindeutige Ablesbarkeit ist auch unter schlechten Sichtbedingungen sichergestellt. Die Höhe der mittleren Axillarlinie des Patienten ist auf einfache Weise einzustellen.

### Ausführungsbeispiel

Anhand eines Ausführungsbeispiels und der Zeichnung soll die Erfindung näher erläutert werden.

In der Zeichnung zeigen:
- Fig. 1:: eine schematische Darstellung einer Einrichtung zur intermittierenden Messung des zentralvenösen Druckes einschließlich der erfindungsgemäßen Bauteile;

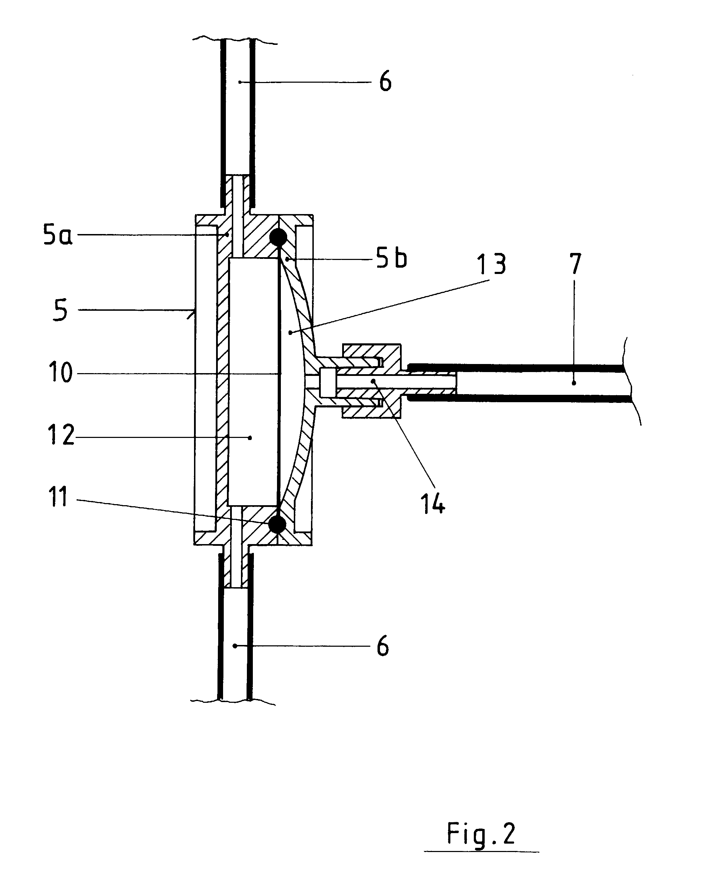

- Fig. 2:: einen Schnitt durch die erfindungsgemäße Druckdose.

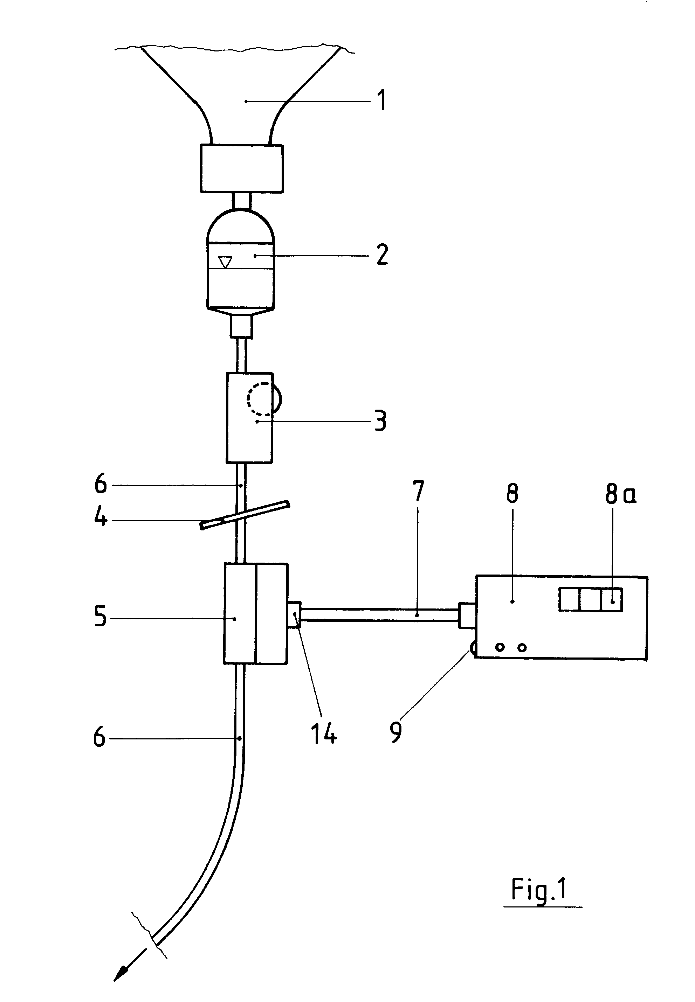

In Fig. 1 ist der übliche Aufbau einer Infusionsanordnung mit der Messung des zentralen Venendruckes ersichtlich. Aus einer Infusionsflasche 1 und einer angeschlossenen Tropfkammer 2 mit darunter befindlicher Rollenklemme 3, mit der die Infusionsmenge bzw. - geschwindigkeit einstellbar ist, gelangt die Infusionsflüssigkeit über eine Infusionsleitung 6 und eine Schiebeklemme 4 in die erfindungsgemäße Druckmeßdose 5 und danach weiter über die Infusionsleitung 6 in die Hohlvene des Patienten. Die Druckmeßdose 5 ist als sogenanntes Schlauchsegment ausgebildet und kann somit in jede beliebige Infusionsanordnung eingeschaltet werden. Sie besitzt dazu einen Eingangs- und einen Ausgangsstutzen für die Infusionsleitung 6 und einen Schlauchanschlußstutzen 14 für den Anschluß einer Schlauchleitung 7 zum Druckdom eines nicht dargestellten Druckwandlers zum elektronischen Meßinstrument 8. Die Druckmeßdose 5 besteht aus zwei Halbschalen 5a und 5b. Der Hohlraum innerhalb dieser Halbschalen 5a und 5b wird durch eine sehr weiche Membran 10 mit einer kräftigen Dichtwulst 11, die eine gute Dichtung gewährleistet, in einen Infusionsraum 12 und einen Luftraum 13 geteilt. Die Halbschale 5b ist innerhalb des Luftraumes 13 als kugelabschnittartige Wölbung mit einem zentral abgehenden Schlauchanschlußstutzen 14 ausgebildet. Wird der Infusionsfluß im Fall einer Venendruckmessung proximal der Druckmeßdose 5 durch die Schiebeklemme 4 unterbrochen, stellt sich im Infusionsraum 12 ein Druck ein, der mit dem Venendruck korrespondiert. Die Einstellung der Rollenklemme 3 wird dadurch nicht verändert. In dem Fall, daß die Mittellinie der Druckmeßdose 5 mit der mittleren Axillarlinie des Patienten auf einem Höhenniveau liegt, was durch eine einfache Vorrichtung eingestellt werden kann, sind Venendruck und Druck im Infusionsraum 12 identisch. Dieser Druck bewirkt eine Wölbung der Membran 10 in den Luftraum 13, die zu einer Verdrängung der Luft dieses Raumes in Richtung der Schlauchleitung 7 mit angeschlossenem elektronischen Meßinstrument 8 mit integrierter digitaler Anzeige 8a führt. Bei einem Unterdruck kehren sich diese Verhältnisse um und es wird ein negativer Druckwert angezeigt. Die richtige Abstimmung von Luftraum 13, Schlauchleitung 7 und elektronischem Meßinstrument 8 führt zur Anzeige des Venendruckes an der digitalen Anzeige 8a. Eine feste Verbindung des elektronischen Meßinstruments 8 mit einer Lichtquelle 9 (z. B. Laserstrahl) und der Druckmeßdose 5 ermöglicht eine einfache Justierung dieses Systems auf dem Niveau der mittleren Axillarlinie des Patienten.. Nach Ablesen und Registrieren dieses Druckes für diagnostische und / oder therapeutische Zwecke wird die Schiebeklemme 4 geöffnet und nach kurzer Anlaufzeit stellt sich der Ursprungszustand der Flüssigkeitszufuhr zum Patienten selbständig wieder ein.

### Aufstellung der verwendeten Bezugszeichen

- 1: Infusionsflasche
- 2: Tropfkammer
- 3: Rollenklemme
- 4: Schiebeklemme
- 5: Druckmeßdose
- 5a: Halbschale
- 5b: Halbschale
- 6: Infusionsleitung
- 7: Schlauchleitung
- 8: elektronisches Meßinstrument
- 8a: digitale Anzeige
- 9: Lichtquelle
- 10: Membran
- 11: Dichtwulst
- 12: Infusionsraum
- 13: Luftraum
- 14: Schlauchanschlußstutzen

## Patentansprüche

1. Einrichtung zur intermittierenden Messung des zentralvenösen Druckes bei gleichzeitiger Einleitung von Infusionsflüssigkeiten in die obere Hohlvene des Menschen, dadurch gekennzeichnet,
daß in eine Infusionsleitung (6) eine Druckmeßdose (5) integriert ist, die aus zwei Halbschalen (5a und 5b), mit dazwischen angeordneter, sehr weicher Membran (10) besteht und daß die Halbschale(5b),die durch die Membran (10) von der mit der Infusionsflüssigkeit durchflossenen Halbschale (5a) getrennt ist, eine kugelabschnittartige Wölbung mit einem zentral abgehenden Schlauchanschlußstutzen (14) aufweist.

2. Einrichtung nach Anspruch 1, dadurch gekennzeichnet,
daß an die Druckmeßdose (5) über den Schlauchanschlußstutzen (14) und einen Schlauch (7) ein elektronisches Meßinstrument (8) mit digitaler Anzeige (8a) angeschlossen ist.

3. Einrichtung nach Anspruch 1 und 2, dadurch gekennzeichnet,
daß zur Justierung der Druckmeßdose (5) auf das Höhenniveau der Hohlvene des zu behandelnden Menschen, die Druckmeßdose (5), das elektronische Meßinstrument (8) mit digitaler Anzeige (8a) und eine einen markierenden Lichtstrahl erzeugende Lichtquelle (9), beispielsweise eine an sich bekannte Laserlichtquelle, zu einer mechanischen Einheit verbunden sind.

4. Einrichtung nach Anspruch 1 bis 3, dadurch gekennzeichnet,
daß während der intermittierenden Messungen des zentralvenösen Druckes, eine Schiebeklemme (4) den Infusionsfluß unterbricht, die vor der Druckmeßdose (5) in die Infusionsleitung (6) eingeschaltet ist.
